# EUROPEAN PATENT SPECIFICATION

(11) **EP 2 297 370 B1**
(45) Date of publication and mention of the grant of the patent: **04.12.2013**
(21) Application number: 08874499.0
(22) Date of filing: 24.12.2008
(51) Int. Cl.: C22C 14/00, C22F 1/18

(54) **BETA-BASED TITANIUM ALLOY WITH LOW ELASTIC MODULUS**
TITANLEGIERUNG AUF BETA-BASIS MIT NIEDRIGEM ELASTIZITÄTSMODUL
ALLIAGE DE TITANE BÊTA AVEC UN FAIBLE MODULE D'ÉLASTICITÉ

(30) Priority: 28.05.2008 KR 20080049737
(43) Date of publication of application: 23.03.2011
(73) Proprietor: Korea Institute Of Machinery & Materials, Daejeon 305-343 (KR)
(72) Inventor: LEE, Dong Geun, Changwon-si Gyeongsangnam-do 641-010 (KR); LEE, Yong Tae, Changwon-si Gyeongsangnam-do 641-010 (KR); MI, Xujun, Beijing (CN); YE, Wenjun, Beijing (CN); HUI, Songxiao, Beijing (CN)
(74) Representative: Beck & Rössig European Patent Attorneys
(86) International application number: PCT/KR2008/007693
(87) International publication number: WO 2009/145406

(56) References cited:
- JP-A- 8 299 428
- JP-A- 2006 089 825
- US-A- 5 871 595
- US-B1- 6 767 418
- SCHNEIDER SERGIO ET AL: "Study of the non-linear stress-strain behavior in Ti-Nb-Zr alloys", MATERIALS RESEARCH, DOT EDITORAÇÃO ELETRÔNICA, SAO CARLOS, BR, vol. 8, no. 4, 1 October 2005 (2005-10-01) , pages 435-438, XP009152292, ISSN: 1516-1439
- Talling: "Deformation of Ti-36Nb-2Ta-3Zr-0.3O (GumMetal) and Applications toMorphing Wings", 2nd SEAS DTC Technical Conference - Edinburgh 2007, 1 January 2007 (2007-01-01), XP55007406, Retrieved from the Internet: URL:http://www.seasdtc.com/events/2007_con ference/papers/PPEM011.pdf [retrieved on 2011-09-16]
- QAZI J I ET AL: "HIGH-STRENGTH METASTABLE BETA-TITANIUM ALLOYS FOR BIOMEDICAL APPLICATIONS", J O M, SPRINGER NEW YORK LLC, UNITED STATES, vol. 56, no. 11, 1 November 2004 (2004-11-01), pages 49-51, XP001243431, ISSN: 1047-4838, DOI: 10.1007/S11837-004-0253-9
- QAZI, J.I. ET AL.: 'High-Strength Metastable Beta-Titanium Alloys for Biomedical Applications' JOM vol. 56, no. 11, November 2004, pages 49 - 51, XP001243431

## Description

The present disclosure relates to a titanium alloy with a low elastic modulus, including no elements harmful to the human body, and more particularly, to a beta-based titanium alloy with a low elastic modulus, including titanium (Ti), niobium (Nb) and zirconium (Zr), and further including tantalum (Ta), hafnium (Hf), molybdenum (Mo), tin (Sn), and the like.

Titanium is widely used in the fields of aerospace, weaponry, nuclear power, sports and leisure, biomedicine and the like due to its high specific strength (strength / weight), high corrosion resistance, excellent mechanical properties including high temperature properties, and excellent biocompatibility.

Biomedical metals have been developed for use in implants for replacing bones, joints, teeth, and the like. The biomedical metals are used for manufacturing a variety of prostheses such as artificial bones, artificial joints, and dental prostheses. Accordingly, biomedical metals should be excellent in biocompatibility as well as mechanical properties, corrosion resistance, and chemical resistance. That is, biomedical metals should be non-toxic and not induce allergies in the human body.

Titanium and titanium alloys have been used as biomaterial for replacing stainless steel. In the beginning, pure titanium and titanium alloy such as Ti-6Al-4V were used as biomaterial.

However, since it came to light that aluminum can cause Alzheimer's disease and vanadium is cytotoxic in the human body, unceasing efforts have been made to develop a new biocompatible alloy based on titanium.

Widely known examples of biocompatible titanium alloys that have been developed to solve the problem of cytotoxicity are Ti-6Al-7Nb and Ti-5Al-2.5Fe, which are second-generation titanium alloys.

Since the 1990s, the problem of "stress shield effect" was newly raised. The stress shield effect is caused by elastic modulus difference between natural bone with a low elastic modulus and biocompatible material with a high elastic modulus.

For example, a metal implant with a high elastic modulus bears most of the load applied to the region around the implant, and the natural bone in the region does not bear any tension, compression and bending for a long time. As a result, the thickness and the weight of the natural bone are reduced gradually, causing serious problems such as osteoporosis around the implant. This phenomenon is referred to as the "stress shield effect." As the natural bone weakens and the density of the osseous tissue of the cortex decreases, the bonding strength between the natural bone and the artificial implant also decreases, resulting in decreased service life of the implant.

As a result, a demand arose for a biomedical metal that satisfies biomechanical suitability requirements as well as bio-chemical suitability requirements including cytotoxicity. That is, a demand emerged for a metal that is not harmful to the human body and has an elastic modulus as low as bones of the human body.

Ti-13Nb-13Zr (ASTM F1713), Ti-12Mo-6Zr-2Fe (ASTM F1813), Ti-15Mo (ASTM F2066), and the like have been developed throughout the world to solve the above mentioned problems. In addition, a variety of alloys such as Ti-35Nb-5Ta-7Zr and Ti-16Nb-13Ta-4Mo in a similar composition range are being developed.

Schneider, Sergio et al disclose in "Study of the non-linear stress-strain behaviour in Ti-Nb-Zr alloys", Materials Research, Vol. 8, No. 4, pages 435-438, 2005 a Ti-41.1Nb-7.1Zr alloy having an elastic modulus of 64.7 GPa.

However, the titanium alloys hitherto developed have an elastic modulus of approximately 60 GPa to approximately 80 GPa, which is still much higher than the elastic modulus of natural bones that range from approximately 10 GPa to approximately 30 GPa. Accordingly, the problem of "stress shield effect" has not been completely solved yet. Therefore, there is a considerable demand for a material that is not harmful to the human body and, at the same time, has a lower elastic modulus. Considering that an alloying element of a high melting point may make the alloying process more difficult and increase the manufacturing cost, the ease of the fabrication process should be taken into account in the development of a titanium alloy.

The present invention aims at providing a titanium alloy composition that is not harmful to the human body, has an elastic modulus as low as bones of the human body, and at the same time, is melted and cast easily and cost- effectively.

This technical problem is solved by the alloys as indicated in the claims.

Noting that a beta phase generally has a low elastic modulus, the inventors selected alloying elements of titanium alloy on the basis of whether they can serve as a beta stabilizer in titanium alloy to lower the elastic modulus of titanium alloy.

Also, the inventors selected the alloying elements of titanium alloy on the basis of whether they are harmless to the human body in terms of biochemical suitability, and whether the density, melting temperature and boiling temperature thereof are economically suitable when compared to titanium. Resultantly, as beta stabilizers satisfying the above requirements, niobium (Nb) and zirconium (Zr) were selected.

Then, the inventors designed a titanium alloy composition having a low elastic modulus using a semi-experimental method for designing and developing an alloy. The method includes calculating the covalent bond order and the energy level of the electrons according to the content of each alloying element, using the electronic state, which is the core of the discrete variational (DV)-Xa molecular orbital method.

Most properties of a material are determined by the electronic state of the material except when a nuclear reaction is involved. Based on the electronic state determining micro-properties of the material on an atomic scale, we can estimate the macro-properties of the material by performing statistical-mechanical analysis. Here, the micro-properties of the material can be analyzed approximately from the electronic state of the material by interpreting the Schrodinger equation and the like.

The inventors calculated the bonding order, Bₒ and the energy level of the electrons, M_{d} of the above-described alloying elements through the DV-Xa molecular orbital method, and discovered a beta-based titanium alloy composition with a low elastic modulus from there-among.

The titanium alloy of the present invention is as defined in the claims. The titanium alloy has an elastic modulus of 55 GPa or lower.

As such, it is possible to realize a low elastic modulus of 55 GPa or lower, which is difficult to realize in a related art Ti-Nb-Zr ternary alloy and a related art quaternary alloy further including another element such as Ta.

Niobium (Nb), which is a major alloying element in the titanium alloy in accordance with the exemplary embodiment, is a soft, grey, ductile metal. Niobium is known as a biocompatible metal because it is stable and does not undergo toxic reactions with fiber cells, corrosion products, and bio-solutions in the human body. In addition, niobium is very stable at room temperature, and has very high corrosion resistance so that it is not corroded by oxygen and strong acids. Niobium is contained in the titanium alloy in a weight percentage ranging from 38 wt.% to 40 wt.% or 37-39 wt.%. This is because the beta phase is difficult to form sufficiently outside this composition range, and thus the elastic modulus increases considerably to 70 GPa or higher.

Zirconium (Zr) has very high corrosion resistance in hot water under acidic or basic atmosphere. Zirconium forms oxide film even in air, showing high corrosion resistance. Zirconium is a biocompatible metal without the cytotoxic effect. Zirconium is contained in the titanium alloy in a range from 5 wt.% to 7 wt.%. This is because the elastic modulus of the ternary alloy of titanium, niobium and zirconium increases considerably outside this range, so that it cannot be applied to a living body.

According to an exemplary embodiment, it is possible to lower the elastic modulus of the titanium alloy to 50 GPa or lower', as well as to 55 GPa or lower.

According to use, one or more elements selected from tantalum (Ta), hafnium (Hf), molybdenum (Mo), and tin (Sn) may be further added in the titanium alloy in a range of 3 wt.% or lower. It is preferable that they are added in a range from 1 wt.% to 3 wt.% in view of the elastic modulus factor. In this case, the content of niobium is from 37 wit.% to 39 wt.%, and the content of zirconium is from 5 wt.% to 7 wt.%.

Tantalum (Ta) is ductile, and has high mechanical strength even at high temperature. Tantalum forms a stable film with high electric resistance so that it is relatively free from oxidation in air. In addition, tantalum is highly resistant to acid, and has excellent compatibility with the human body, so that it can be used for cementing bones. Tantalum, when alloyed in titanium, serves as a major beta stabilizer.

Hafnium (Hf) has characteristics very similar to zirconium, and has excellent corrosion-resistance and bio-compatibility. It serves as a beta stabilizer when alloyed in titanium.

Molybdenum (Mo) has a relatively high melting point. However, it has excellent thermal conductivity, high corrosion resistance even in strong acid, and very favorable mechanical properties over a wide temperature range. It serves as a beta stabilizer when alloyed in titanium.

Tin (Sn) is stable in an air and has excellent ductility. It is soluble in acids and alkalis, and has a very low melting temperature of about 232 °C. It is stable in the human body and thus widely used in the fields of table ware, plating and the like. It may also serve as a beta stabilizer when alloyed in titanium.

Addition of the above elements in an amount greater than 3 wit.% may affect the titanium-niobium-zirconium ternary system to increase the elastic modulus. Accordingly, the maximum content of the above-mentioned elements in the titanium alloy is set to 3 wt.% or lower.

The titanium alloy in accordance with the exemplary embodiments can be fabricated by various melting or casting methods such as vacuum induction melting (VIM), vacuum arc remelting (VAR), induction skull melting (ISM), plasma arc melting (PAM), electron beam melting (EBM) and the like.

The beta-based titanium alloy in accordance with the exemplary embodiments of the present invention has low elastic modulus and excellent mechanical properties. Therefore, it can be used in a variety of applications, for example, as a material for medical devices, such as artificial bones, artificial teeth and artificial hip joints, as a material for general civilian goods such as eyewear frames and headsets, and as a material for sports and leisure goods such as golf clubs.
FIG. 1 is a photograph of an ingot prepared by melting and casting a titanium alloy in accordance with an exemplary embodiment.
FIG. 2 is a photograph of a cylinder-shaped product prepared by drawing the ingot of FIG.1.
FIGS. 3 and 4 are micrographs, each showing a microstructure of a titanium alloy in accordance with Embodiment 1.
FIG. 5 is a micrograph showing a microstructure of a titanium alloy in accordance with Embodiment 2.

Hereinafter, specific embodiments will be described in detail with reference to the accompanying drawings. However, it should be understood that the description of the embodiment is merely illustrative and should not be taken in a limiting sense.

### Embodiment 1

Ti-Nb-Zr ternary alloys having compositions as listed in Table 1 were prepared by a vacuum arc remelting (VAR) process.

In order for uniform alloy composition, process convenience, process economy, time and energy savings and the like, a Ti-Nb master alloy was used to cast beta-based titanium alloys.

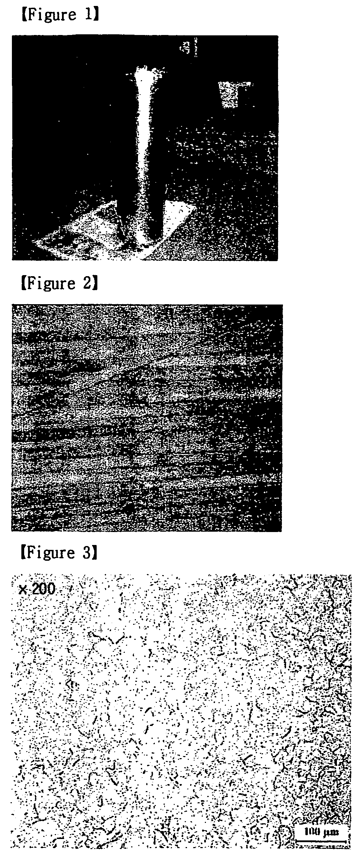

The titanium alloys melted by the VAR process in accordance with the embodiment were cast into ingots as shown in Fig. 1. Then, the ingots were processed into bars having a diameter of 15 mm as shown in Fig. 2, through a drawing process.

The ingot had an excellent appearance. Surface crack, fracture and the like that are often generated during the drawing process were not observed in the surface of the bar. Accordingly, it can be concluded that the titanium alloys in accordance with the embodiment have good formability and good workability.

The alloy bar fabricated in accordance with the embodiment was cut into a section perpendicular to the drawing direction and a section parallel to the drawing direction. The cut surface was first macro-polished with abrasive papers of up to 2400 grit and then micro-polished with a diamond paste.

After the mechanical polishing, the cut surface was etched with Kroll etchant (H₂O 100 ml + HNO₃ 5 ml + HF 3 ml) and then the microstructure of the cut surface was observed using an optical microscope.

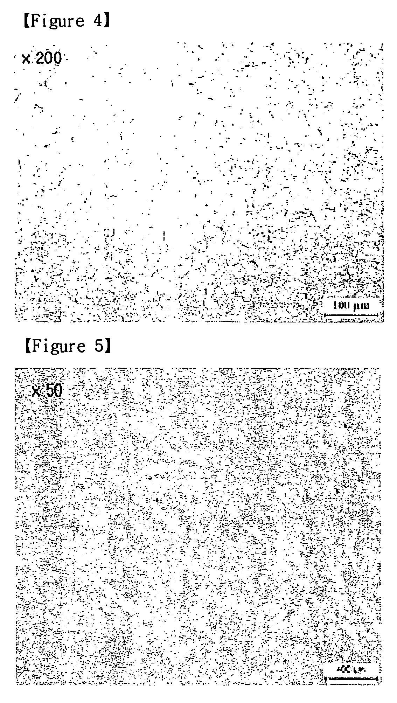

FIG. 3 is a micrograph (at 200x magnification) of a surface of the specimen No. 4 (Table 1) cut perpendicular to the drawing direction. FIG. 4 is a micrograph (at 200x magnification) of a surface of the specimen No. 4 (Table 1) cut parallel to the drawing direction. Referring to FIGS. 3 and 4, the beta-based titanium alloy fabricated in accordance with the embodiment had uniform grain size, and showed no segregations and no defects.

Four test specimens were taken from the titanium alloy in accordance with the embodiment. Then, an elastic compression test was performed according to ASTM E9-89a specifications. The average elastic moduli of the specimens obtained from the elastic compression test are given in Table 1.

**Table 1**

| Specimen No. | Composition of alloy (wt.%) | Elastic modulus (GPa) | Remarks |
|---|---|---|---|
| 1 | Ti-34Nb-11Zr | 68 | Comparative |
| 2 | Ti-35Nb-8.2Zr | 72 | Comparative |
| 3 | Ti-37,9Nb-7.4Zr | 41.5 | Experimental |
| 4 | Ti-38.9Nb-5.5Zr | 38.9 | Experimental |
| 5 | Ti-39Nb-6Zr | 40 | Experimental |
| 6 | Ti-40.9Nb-5Zr | 40 | Experimental |
| 7 | Ti-42.4Nb-5.5Zr | 74 | Comparative |
| 8 | Ti-43Nb-12Zr | 81 | Comparative |

As can be seen from the measured elastic modulus data in Table 1, contents of niobium and zirconium in the comparative examples were similar to those in the Experimental examples. However, the elastic moduli of the comparative examples were 80 % to 100 % greater than those of the experimental examples.

That is, by minimizing the addition of the alloying elements through a new alloy design for restricting the amount of the alloying elements, the ternary titanium alloy in accordance with the embodiment can achieve the ultra-low elastic modulus, which has been difficult to achieve even in related art quaternary titanium alloys.

### Embodiment 2

Contrary to Embodiment 1, a titanium alloy in accordance with Embodiment 2 further includes tantalum (Ta) as shown in Table 2, so as to improve mechanical properties while still maintaining the low elastic modulus and including no elements harmful to the human body. The titanium alloys were melted by the vacuum arc remelting (VAR) process, cast into ingots, and then drawn into bars, as described in Embodiment 1.

Specimens were cut from the alloy bars and polished mechanically. After etching the specimen, the microstructure was observed at a magnification of 50x using an optical microscope. As shown in FIG. 5, there were no segregations and no defects visible in the microstructure of the alloy.

Further, the elastic compression test was performed four times according to ASTM E9-89a specifications to measure the elastic modulus of the alloy. The average elastic moduli of the specimens obtained from the elastic compression test are given in Table 2.

**Table 2**

| Specimen No. | Composition of alloy (wt.%) | Elastic modulus (GPa) | Remarks |
|---|---|---|---|
| 9 | Ti-37.3Nb-5.8Zr-2.9Ta | 43 | Experimental |
| 10 | Ti-39Nb-6.5Zr-1.5Ta | 39 | Experimental |

As shown in Table 2, the titanium alloys in accordance with Embodiment 2 were not increased in the elastic modulus in comparison with the titanium alloys in accordance with Embodiment 1. Accordingly, the titanium alloy in accordance with Embodiment 2 can be used to achieve the required mechanical properties as well as the elastic modulus.

## Claims

1. A titanium alloy consisting of:
38 wt.% to 40 wt.% niobium (Nb),
5 wt.% to 7 wt.% zirconium (Zr), and
optionally one or more elements selected from tantalum (Ta), hafnium (Hf), molybdenum (Mo) and tin (Sn), whose total content is 3 wt.% or lower,
with the balance being titanium(Ti),
wherein the elastic modulus of the titanium alloy is 55 GPa or lower.

2. The titanium alloy of claim 1, wherein the elastic modulus thereof is 50 GPa or lower.

3. A titanium alloy consisting of:
37wt.% to 39wt.% niobium (Nb),
5wt.% to 7wt.% zirconium (Zr),
1 wt.% to 3wt.% tantalum (Ta),
with the balance being titanium(Ti),
wherein the elastic modulus thereof is 50 GPa or lower.

## Patentansprüche

1. Titanlegierung, bestehend aus:
38 % bis 40 % Gewichtsprozent Niob (Nb),
5 % bis 7 % Gewichtsprozent Zirkon (Zr), und
optional einem oder mehreren Elementen aus Tantal (Ta), Hafnium (Hf), Molybdän (Mo) und Zinn (Sn), deren Gesamtanteil 3 % Gewichtsprozent oder weniger ist,
wobei der Rest Titan (Ti) ist,
wobei der Elastizitätsmodul der Titanlegierung 55 GPa oder weniger ist.

2. Titanlegierung nach Anspruch 1, bei der der Elastizitätsmodul derselben 50 GPa oder weniger ist.

3. Titanlegierung, bestehend aus:
37 % bis 39 % Gewichtsprozent Niob (Nb),
5 % bis 7 % Gewichtsprozent Zirkon (Zr),
1 % bis 3 % Gewichtsprozent Tantal (Ta),
wobei der Rest Titan (Ti) ist,
wobei der Elastizitätsmodul derselben 50 GPa oder weniger ist.

## Revendications

1. Alliage de titane consistant en
38 % en poids à 40 % en poids de niobium (Nb),
5 % en poids à 7 % en poids de zirconium (Zr) et
de manière optionnelle un ou plusieurs éléments sélectionnés parmi le tantale (Ta), le hafnium (Hf), le molybdène (Mo) et l'étain (Sn) dont la teneur totale est de 3 % en poids ou moins,
avec le complément en titane (Ti),
dans lequel le module élastique de l'alliage de titane est 55 GPa ou moins.

2. Alliage de titane selon la revendication 1 dans lequel le module élastique de celui-ci est 50 GPa ou moins.

3. Alliage de titane consistant en
37 % en poids à 39 % en poids de niobium (Nb),
5 % en poids à 7 % en poids de zirconium (Zr),
1 % EN poids à 3 % en poids de tantale (Ta),
avec le complément en titane (Ti),
dans lequel le module élastique de l'alliage de titane est 50 GPa ou moins.
